(19) Europäisches Patentamt
European Patent Office
Office européen des brevets

(11) Veröffentlichungsnummer: **0 027 913 A2**

(12) # EUROPÄISCHE PATENTANMELDUNG

(21) Anmeldenummer: **80105919.7**

(22) Anmeldetag: **30.09.80**

(51) Int. Cl.³: **B 41 M 5/12**, B 41 M 5/26

(30) Priorität: **26.10.79 CH 9629/79**
**26.10.79 CH 9630/79**

(43) Veröffentlichungstag der Anmeldung: **06.05.81**
**Patentblatt 81/18**

(84) Benannte Vertragsstaaten: **BE CH DE FR GB LI**

(71) Anmelder: **CIBA-GEIGY AG, Patentabteilung Postfach, CH-4002 Basel (CH)**

(72) Erfinder: **Petitpierre, Jean Claude, Dr., Aeussere Reben 322, CH-4303 Kaiseraugst (CH)**

(74) Vertreter: **Zumstein sen., Fritz, Dr. et al, Bräuhausstrasse 4, D-8000 München 2 (DE)**

(54) **Druckempfindliches oder wärmeempfindliches Aufzeichnungsmaterial.**

(57) Ein druckempfindliches oder wärmeempfindliches Aufzeichnungsmaterial, welches in seinem farbbildenden System als Farbentwickler für den Farbbildner mindestens eine Verbindung der Formel

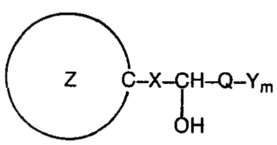

(1)

enthält, worin der Ring Z einen heterocyclischen Rest darstellt, welcher benachbart zum Bindungskohlenstoffatom keine Ketogruppe aufweist, X die direkte Bindung –O–, –CHR–, –NR–, –CONR–, $-SO_2NR-$, $-NR_1-CO-NR_2-$, $-NR_1-CS-NR_2-$ oder $-NR_1-SO_2-NR_2-$, R, $R_1$ und $R_2$ unabhängig voneinander Wasserstoff, unsubstituiertes oder durch Halogen, Cyano oder Niederalkoxy substituiertes Alkyl mit höchstens 12 Kohlenstoffatomen, Benzyl, Phenyl, oder durch Halogen, Methyl oder Methoxy substituiertes Benzyl oder Phenyl, Q Kohlenstoff oder einen unsubstituierten oder substituierten Kohlenwasserstoffrest, Y Halogen und m 1 bis 3 bedeuten.

Die Gruppierung $-Q-Y_m$ ist vorzugsweise $-C(Hal)_3$, worin Hal Halogen bedeutet.

EP 0 027 913 A2

ACTORUM AG

CIBA-GEIGY AG
Basel (Schweiz)

1-12568/69

Druckempfindliches oder wärmeempfindliches Aufzeichnungsmaterial

Die vorliegende Erfindung betrifft ein druckempfindliches oder wärmeempfindliches Aufzeichnungsmaterial, welches in seinem Farbreaktantensystem als Farbentwickler für den Farbbildner mindestens eine Verbindung der Formel

(1) 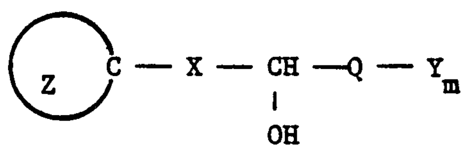

enthält, worin der Ring Z einen heterocyclischen Rest darstellt, welcher benachbart zum Bindungskohlenstoffatom keine Ketogruppe aufweist, X die direkte Bindung, -O-, -CHR-, -NR-, -CONR-, $-SO_2NR-$, $-NR_1-CO-NR_2-$, $-NR_1-CS-NR_2-$ oder $-NR_1-SO_2-NR_2-$, R, $R_1$ und $R_2$ unabhängig voneinander Wasserstoff, unsubstituiertes oder durch Halogen, Cyano oder Niederalkoxy substituiertes Alkyl mit höchstens 12 Kohlenstoffatomen, Benzyl, Phenyl, oder durch Halogen, Methyl oder Methoxy substituiertes Benzyl oder Phenyl, Q Kohlenstoff oder einen unsubstituierten oder substituierten Kohlenwasserstoffrest, Y Halogen und m 1 bis 3 bedeuten.

Unter den Verbindungen der Formel (1) sind diejenigen, in denen Q Kohlenstoff und m 3 sind, bevorzugt.

Z stellt ein heterocyclisches Ringsystem dar, welches über ein Ringkohlenstoffatom mit X verbunden ist und welches benachbart zu diesem Bindungskohlenstoffatom keine Ketogruppe (CO) aufweist. Der heterocyclische Rest Z kann ein- oder mehrkernig sein und 5 bis 15, vorzugsweise 5 bis 10 Ringglieder aufweisen, wobei 1 bis 3, vorzugsweise 1 oder 2 Heteroatome als Ringglieder vorhanden sein können.

Bevorzugt stellt Z einen von zum Bindungskohlenstoffatom benachbarten Ketogruppen freien, fünf- oder sechsgliedrigen Heterocyclus von aromatischem Charakter dar, der als Ringglieder vorzugsweise Sauerstoff, Schwefel und/oder Stickstoff enthält. Beispiele derartiger Heterocyclen sind Thienyl-, Furyl-, Pyrrolyl-, Pyrazolyl-, Imidazolyl-, Thiazolyl-, Pyridyl-, Pyrimidyl-, Thiadiazolyl-, Triazolyl- oder Isothiazolylreste.

Dabei kann Z auch ein mehrkerniges z.B. zwei- oder dreikerniges, heterocyclisches Ringsystem darstellen. Dieses enthält vorzugsweise einen dem Heterozyklus ankondensierten Benzol- oder Naphthalinring, wie z.B. ein gegebenenfalls substituierter Benzofuran-, Benzothiophen-, Indol-, Indazol-, Benzimidazol-, Benzothiazol-, Benzoisothiazol-, Benzotriazol-, Naphthotriazol-, Chinolin- oder Carbazolylrest.

Die ein- oder mehrkernigen Z-Reste können ein- oder mehrmals, gleich oder verschieden z.B. durch Halogen, Cyano, Nitro, Hydroxyl, Niederalkyl, Niederalkoxy, Niederalkylcarbonyl, Niederalkoxycarbonyl, Niederalkylamino, Diniederalkylamino, N-Niederalkylcarbamyl, N,N-Diniederalkylcarbamyl oder Phenyl substituiert sein.

Die definitionsgemässen heterocyclischen Reste Z können auch nicht-aromatisch sein. Beispiele derartiger Substituenten sind die den oben aufgeführten aromatischen Heterocyclen entsprechenden, teilweise oder vollständig hydrierten heterocyclischen Reste, die gegebenenfalls auch substituiert, z.B. durch Niederalkyl, sein können.

X bedeutet vorzugsweise die direkte Bindung, -CHR- oder -NR-. X kann jedoch vorteilhafterweise auch -O-, -CONR-, $-SO_2NR-$, $-NR_1-CO-NR_2-$ oder $-NR_1-SO_2-NR_2-$ darstellen.

R, $R_1$ und $R_2$ sind vorzugsweise Wasserstoff. Stellen die Substituenten R, $R_1$ und $R_2$ Alkylgruppen dar, so können sie geradkettig oder verzweigt sein. Beispiele für solche Alkylreste sind Methyl, Aethyl, n-Propyl, Isopropyl, n-Butyl, sek.-Butyl, n-Hexyl, n-Octyl oder n-Dodecyl. Sind die Alkylreste in R, $R_1$ und $R_2$ substituiert, so handelt es sich vor allem um Cyanoalkyl, Halogenalkyl oder Alkoxyalkyl jeweils mit insgesamt 2 bis 5 Kohlenstoffatomen wie z.B. β-Cyanoäthyl, β-Chloroäthyl, β-Methoxyäthyl oder β-Aethoxyäthyl.

Bevorzugte Substituenten in der Benzyl- und Phenylgruppe der R-, $R_1$- und $R_2$-Reste sind z.B. Halogen, Methyl oder Methoxy.

Q kann ein aliphatischer, cycloaliphatischer, aliphatischaromatischer oder aromatischer Kohlenwasserstoffrest sein, der gegebenenfalls substituiert ist. In der Bedeutung eines aliphatischen Restes stellt Q vorteilhafterweise einen $C_1-C_6$-Alkylenrest, insbesondere einen $C_1-C_4$-Alkylenrest dar, der geradkettig oder verzweigt und durch Halogen, Carboxyl, $-SO_3H$, Phenyl oder Halogenphenyl substituiert sein kann. Als cycloaliphatischer Rest kommt vor allem die Cyclohexylengruppe in Frage. Als aromatischer Rest bedeutet Q vorzugsweise Diphenylen oder in erster Linie Phenylen, die durch Halogen, Carboxyl, $-SO_3H$, Niederalkyl oder Niederalkoxy substituiert sein können.

Niederalkyl und Niederalkoxy stellen bei der Definition der Reste in der Regel solche Gruppen oder Gruppenbestandteile dar, die 1 bis 5, insbesondere 1 bis 3 Kohlenstoffatome aufweisen, wie z.B. Methyl, Aethyl, n-Propyl, Isopropyl, n-Butyl, sek-Butyl, tert-Butyl, oder Amyl bzw. Methoxy, Aethoxy oder Isopropoxy.

Halogen in Verbindung mit den Substituenten der Verbindungen der Formel (1) ist beispielsweise Fluor, Brom oder vorzugsweise Chlor.

Bei der Formel (1) bedeutet X in erster Linie eine -NH-Gruppe und vor allem die -NH-CO-NH-Gruppe.

Praktisch wichtige Farbentwickler der Formel (1) entsprechen der Formel

(2) 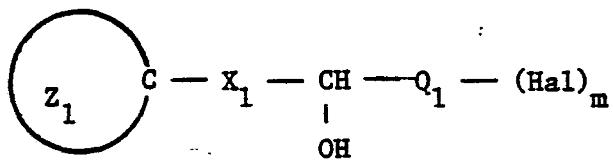

worin der Ring $Z_1$ einen unsubstituierten oder durch Halogen, Cyano, Hydroxyl, Niederalkyl, Niederalkoxy, Niederalkylcarbonyl, Niederalkylamino, Diniederalkylamino, N-Niederalkylcarbamyl, N,N-Diniederalkylcarbamyl oder Phenyl substituierten, ein- oder zweikernigen, heterocyclischen Rest darstellt, welcher benachbart zum Bindungskohlenstoffatom keine Ketogruppe aufweist, und $X_1$ die direkte Bindung, -O-, $-CH_2-$, $-CHR_3-$, $-NR_3-$, -CONH-, $-SO_2NH-$, -NH-CO-NH- oder $-NH-SO_2-NH-$, $R_3$ Niederalkyl, $Q_1$ Kohlenstoff, $C_1$-$C_5$ Alkylen oder Phenylen, Hal Fluor, Chlor oder Brom und m 1 bis 3 bedeuten.

Der Ring $Z_1$ weist bevorzugt aromatischen Charakter auf. $R_3$ ist vorzugsweise Methyl. $X_1$ steht insbesondere für -O-, -CONH-, $-SO_2NH-$, -NH-CO-NH- oder $-NH-SO_2-NH-$.

Unter den Verbindungen der Formel (2) sind diejenigen der Formel

(3) 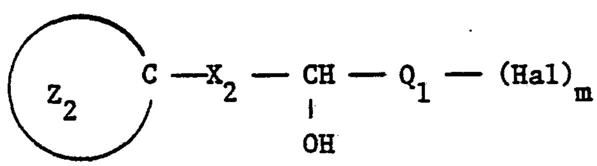

worin der Ring $Z_2$ einen unsubstituierten oder durch Halogen, Cyano, Hydroxyl, Niederalkyl, Niederalkoxy, Niederalkylcarbonyl, N-Niederalkylcarbamyl, N,N-Diniederalkylcarbamyl oder Phenyl substituierten, ein- oder zweikernigen, heterocyclischen Rest darstellt, welcher benachbart zum Bindungskohlenstoffatom keine Ketogruppe aufweist, und $X_2$ die direkte Bindung, $-CH_2-$, $-CHR_3-$ oder -NH- bedeutet und $R_3$, $Q_1$, Hal und m die angegebene Bedeutung haben, bevorzugt.

Besonders bevorzugt als Farbentwickler eingesetzte Verbindungen entsprechen der Formel

(4) 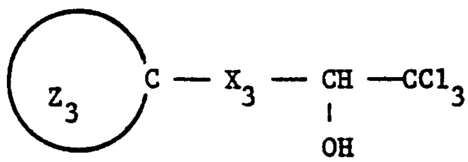

worin der Ring $Z_3$ einen unsubstituierten oder durch Halogen, Cyano, Hydroxyl, Niederalkyl, Niederalkoxy, N-Niederalkylcarbamyl, N,N-Diniederalkylcarbamyl oder Phenyl substituierten Furyl-, Thienyl-, Pyridyl-, Pyrimidyl-, Thiazolyl- oder Chinolinylrest und $X_3$ die direkte Bindung, $-CH_2-$, -O-, -NH-, -CONH- oder -NH-CO-NH- bedeuten. Dabei bedeutet $X_3$ insbesondere die direkte Bindung, $-CH_2-$ oder -NH-. $Z_3$ ist in Formel (4) bevorzugt Pyridyl.

Von grossem Interesse als Farbentwickler sind jedoch Verbindungen der Formel (4), in der $X_3$ -O-, -CONH- oder -NH-CO-NH- und $Z_3$ einen unsubstituierten oder durch Halogen, Cyano, Hydroxyl, Niederalkyl, Niederalkoxy oder Phenyl substituierten Furyl-, Thienyl-, Pyridyl-, Pyrimidyl-, Thiazolyl- oder Chinolinylrest bedeuten.

Im Vordergrund des Interesses stehen Verbindungen der Formel (4), in der $X_3$ -NH-CO-NH- und der Ring $Z_3$ Furyl, Pyridyl, Pyrimidyl oder Chinolinyl bedeuten.

Die erfindungsgemäss eingesetzten Verbindungen der Formeln (1), (2), (3) und (4) sind als Stoffe zum Teil bekannt, stellen jedoch eine neue Klasse von Farbentwicklern oder Elektronen-akzeptoren für Farbbildner dar. Sie können nach an sich bekannten Verfahren hergestellt werden. Beispielsweise kann die Herstellung dadurch erfolgen, dass man ein Mol einer heterocyclischen Verbindung der Formel

(5) 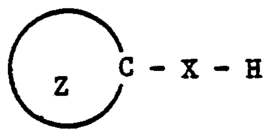

mit einem Aldehyd der Formel

(6) $Y_m - Q - CHO$

oder dessen Hydrat umsetzt, worin Z, X, Y, Q und m die angegebene Bedeutung haben.

Die Umsetzung erfolgt zweckmässig in einem indifferenten Lösungsmittel, gegebenenfalls unter Zusatz geringer Mengen basischer Katalysatoren. Als Lösungsmittel kommen beispielsweise cycloaliphatische oder aromatische Kohlenwasserstoffe wie z.B. Cyclohexan, Benzol, Toluol oder Xylol; Chlorkohlenwasserstoffe wie Chloroform, Aethylenchlorid oder Chlorbenzole; Aether wie Dioxan, Diäthyläther, Glykoldimethyläther oder Tetrahydrofuran sowie Dimethylformamid, Dimethylsulfoxid und Acetonitril. Als basische Katalysatoren eignen sich z.B. Alkylamine, Alkanolamine, Piperidin, Pyridin oder Ammoniumacetat. Die Umsetzung kann bei einer Temperatur von 10 bis 100°C, vorzugsweise 20 bis 80°C vorgenommen werden.

Bekannte Verbindungen der Formeln (1) bis (4) und deren Herstellung werden z.B. in Chemical Reviews 75(1975) 259-289, R. Hull, J. Chem. Soc. 4845 (1957) und B. Hesse, A. Moll, J.f. prakt. Chemie, Band 316, (1974), 304-314 beschrieben.

Bei den Ausgangsstoffen der Formel (5) handelt es sich um definitionsgemässe heterocyclische Verbindungen, die mindestens ein

reaktionsfähiges Ringkohlenstoffatom oder mindestens eine reaktionsfähige $R-CH_2$-gruppe, insbesondere Methylgruppe oder eine reaktionsfähige Hydroxyl-, Amino-, Carbamyl-, Sulfamyl-, Ureido-, Thioureido- oder Aminosulfamoylgruppe aufweisen. Diese Gruppen reagieren mit der Aldehydgruppierung der Verbindungen der Formel (6) und bilden somit die Verbindungen der Formel (1).

Beispiele von als Ausgangsstoffen der Formel (5) verwendbaren heterocyclischen Verbindungen sind Furan, 2-Methylfuran, N,N-Diäthyl-2 furamide, Thiophen, Kollidin, 8-Hydroxychinolin, 8-Aminochinolin, 4-Amino-2-methylchinolin, 2-Phenyl-4-methylchinolin, 3-Amino-indazol, 6-Aminoindazol, 2-Phenyl-4-methyl-6-methoxychinolin, 3-Aminopyridin, 2-Amino-3-chloro-6-methyl-pyrimidin, Chinaldin, 2-Aethylchinolin, 5-Amino-triazol, 5-Amino-pyrazol, 2-Methylthiazol, 2-Amino-benzothiazol, 2-Amino-6-methoxy-benzothiazol und 2-Amino-6-cyanobenzothiazol, ferner Nicotinsäureamid, Isonicotinsäureamid, 3-Pyridylharnstoff, 2-Pyridylharnstoff, 3-Hydroxypyridin, 2-Carbamylfuran, 1-Methyl-3-hydroxy-piperidin, 5-Sulfamylbenzoxazolon, 2-Benzthiazolyl-sulfamid, 1,3-Dimethyl-4-uracilyl-sulfamid, 2-Hydroxy-9-methyl-carbazol, 4-Ureido-phthalsäureanhydrid.

Als Beispiele für als Ausgangsstoffe der Formel (6) dienende Aldehyde seien genannt:
Chloracetaldehyd, Bromacetaldehyd, Fluoracetaldehyd, Trichloracetaldehyd, Tribromacetaldehyd, Trifluoracetaldehyd, Tribrompropionaldehyd, α-Chlorcrotonaldehyd, Trichlorbutyraldehyd, 2,3-Dibrom-3,3-dichlor-propional, 2,2,3-Trichlorpentanal, Trichlorbenzaldehyd, 2,3-Dichlor-3-phenylpropionaldehyd, 2,2,3-Trichlor-3-phenylpropionaldehyd, 2-Chlor-2,3-dibrom-3-phenylpropionaldehyd und 2,2,3-Trichlor-3-(3'-chlorphenyl)-propionaldehyd.

Einen weiteren Gegenstand der vorliegenden Erfindung betreffen die neuen Verbindungen unter den heterocyclischen Verbindungen der Formel (1), welche der Formel

(7) 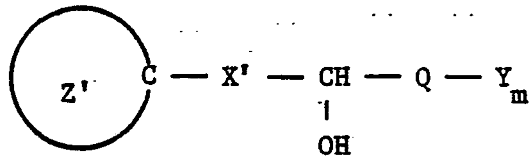

entsprechen, worin der Ring Z' einen heterocyclischen Rest, X' -O-, -CONR-, $-SO_2NR-$, $-NR_1-CO-NR_2-$, $-NR_1-CS-NR_2-$ oder $-NR_1-SO_2-NR_2-$, R, $R_1$ und $R_2$ unabhängig voneinander Wasserstoff, unsubstituiertes oder durch Halogen, Cyano oder Niederalkyl substituiertes Alkyl mit höchstens 12 Kohlenstoffatomen, Benzyl, Phenyl oder durch Halogen, Methyl oder Methoxy substituiertes Benzyl oder Phenyl, Q Kohlenstoff oder einen unsubstituierten oder substituierten Kohlenwasserstoffrest, Y Halogen und m 1 bis 3 bedeuten. Q ist vorzugsweise Kohlenstoff und m ist bevorzugt gleich 3,

X' bedeutet vorzugsweise -O-, -CONR-, $-SO_2NR-$, $-NR_1-CO-NR_2-$ oder $-NR_1-SO_2-NR_2-$ und insbesondere die Gruppe -NH-CO-NH-.

Diese neuen Verbindungen der Formel (7) sind als besonders vorteilhafte Farbentwickler für Farbbildner hervorzuheben.

Die Erläuterungen für die Substituenten Z, X, Q, Y und m der Formel (1) gelten sinngemäss auch für die entsprechenden Reste Z', X', Q, Y und m der Formel (7).

Bevorzugte neue Verbindungen entsprechen der Formel

(8) 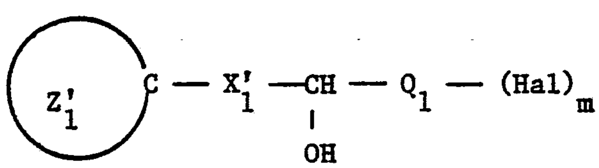

worin der Ring $Z_1'$ einen unsubstituierten oder durch Halogen, Cyano, Hydroxyl, Niederalkyl, Niederalkoxy, Niederalkylcarbonyl, Niederalkylamino, Diniederalkylamino, N-Niederalkylcarbamyl, N,N-Diniederalkylcarbamyl oder Phenyl substituierten, ein- oder zweikernigen heterocyclischen Rest, $X_1'$ -O-, -CONH-, $-SO_2NH-$, -NH-CO-NH- oder $-NH-SO_2-NH-$, $Q_1$ Kohlenstoff, $C_1$-$C_5$-Alkylen oder Phenylen, Hal Fluor, Chlor oder Brom und m 1 bis 3 bedeuten.

Von besonderem Interesse sind Verbindungen der Formel

(9) 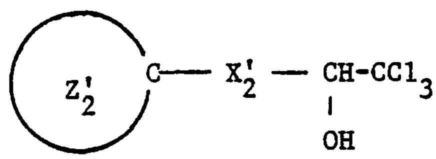

worin der Ring $Z_2'$ einen unsubstituierten oder durch Halogen, Cyano, Hydroxyl, Niederalkyl, Niederalkoxy oder Phenyl substituierten Furyl-, Thienyl-, Pyridyl-, Pyrimidyl-, Thiazolyl- oder Chinolinylrest, und $X_2'$ -O-, -CONH- oder -NH-CO-NH- bedeuten.

Unter diesen Verbindungen der Formel (9) sind diejenigen, in denen der Ring $Z_2'$ Furyl, Pyridyl, Pyrimidyl oder Chinolinyl und $X_2'$ -NH-CO-NH- bedeuten, als Färbentwickler besonders gut geeignet.

Die erfindungsgemässen Verbindungen der Formel (7) werden dadurch hergestellt, dass man eine heterocyclische Verbindung der Formel

(5a) 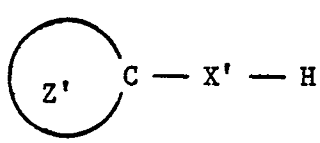

mit einem Aldehyd der Formel

(6a) $Y_m$ —Q—CHO

oder dessen Hydrat umsetzt, worin Z', X', Y, Q und m die angegebene Bedeutung haben.

Auf gleiche Art und Weise werden auch die neuen Verbindungen der Formeln (8) und (9) hergestellt.

Die erfindungsgemäss eingesetzten Verbindungen der Formeln (1) bis (4) sind praktisch farb- und geruchslos und mit den üblichen Farbbildnern sehr reaktiv, so dass damit spontane, beständige und nicht verblassende Aufzeichnungen oder Kopien erhalten werden.

Die im erfindungsgemässen Aufzeichnungsmaterial oder Kopiermaterial in Betracht kommenden Farbbildner sind bekannte farblose oder schwach gefärbte Stoffe, die, sofern sie mit den Verbindungen der Formeln (1) bis (4) in Kontakt kommen, farbig werden oder die Farbe ändern. Farbbildner oder deren Mischungen können verwendet werden, die z.B. den Klassen der Phthalide, Fluorane, Benzofluorane, Spiropyrane, Azomethine, Leukoauramine, Triarylmethan-leukofarbstoffe, Phenoxazine, Phenothiazine sowie der Chromeno- oder Chromanofarbbildner angehören. Als Beispiele solcher geeigneten Farbbildner seien genannt:

Kristallviolettlacton, 3,3-(Bisaminophenyl)-phthalide, 3,3-(Bis-substituierte-Indolyl)-phthalide, 3-(Aminophenyl)-3-Indolyl-phthalide,
6-Dialkylamino-2-n-octylamino-fluorane,
6-Dialkylamino-2-arylamino-fluorane,
6-Dialkylamino-3-methyl-2-arylamino-fluorane
6-Dialkylamino-2- oder 3-niederalkyl-fluorane,
6-Dialkylamino-2-dibenzylamino-fluorane,
Bis-(aminophenyl)-furyl- oder -phenyl- oder -carbazolyl-methane oder Benzoylleukomethylenblau.

Die Verbindungen der Formel (1) eignen sich als Farbentwickler für druck- oder wärmeempfindliches Aufzeichnungsmaterial, das sowohl Kopier- als auch Registriermaterial sein kann.

Ein druckempfindliches Material besteht beispielsweise aus mindestens einem Paar von Blättern, die mindestens einen Farbbildner

gelöst in einem organischen Lösungsmittel und einen Entwickler der Formel (1) enthalten. Der Farbbildner liefert an den Punkten, an denen er mit dem Entwickler in Kontakt kommt, eine gefärbte Markierung.

Die Entwickler der Formel (1) können für sich allein, als Mischungen oder in Mischung mit bekannten Entwicklern eingesetzt werden. Die Entwickler werden vorzugsweise in Form einer Schicht auf die Vorderseite des Empfangsblattes aufgebracht.

Typische Beispiele für bekannte Entwickler sind Attapulgus-Ton, Bentonit, säureaktiviertes Bentonit, Montmorillonit, Halloysit, Siliciumdioxyd, Aluminiumoxid, Aluminiumsulfat, Aluminiumphosphat, Zinkchlorid, Kaolin oder irgendein beliebiger Ton oder sauer reagierende, organische Verbindung, wie z.B. gegebenenfalls ringsubstituierte Phenole, Salicylsäure oder Salicylsäureester und deren Metallsalze, ferner ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolisiertes Polymerisat von Maleinsäureanhydrid mit Styrol, Aethylen oder Vinylmethyläther, oder Carboxypolymethylen.

Um zu verhindern, dass die Farbbildner, die in dem druckempfindlichen Aufzeichnungsmaterial enthalten sind, frühzeitig aktiv werden, werden sie in der Regel von dem Entwickler getrennt. Dies kann zweckmässig erzielt werden, indem man die Farbbildner in schaum-, schwamm- oder bienenwabenartige Strukturen einarbeitet. Vorzugsweise sind die Farbbildner in Mikrokapseln eingeschlossen, die sich in der Regel durch Druck zerbrechen lassen.

Wenn die Kapseln durch Druck, beispielsweise mittels eines Bleistiftes zerbrochen werden und wenn die Farbbildnerlösung auf diese Weise auf ein benachbartes Blatt übertragen wird, das mit dem Entwickler der Formel (1) beschichtet ist, wird eine farbige Stelle erzeugt. Diese Farbe resultiert aus dem dabei gebildeten Farbstoff, der im sichtbaren Bereich des elektromagnetischen Spektrum absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogeniertes Paraffin oder Diphenyl, wie Chlorparaffin oder Trichlordiphenyl, ferner Tricresylphosphat, Di-n-butylphthalat, aromatische Aether, wie Benzylphenyläther, Kohlenwasserstofföle, wie Paraffin oder Kerosin, alkylierte Derivate von Diphenyl, Naphthalin oder Triphenyl, Dibenzyltoluol, Terphenyl, partiell hydriertes Terphenyl oder weitere chlorierte oder hydrierte, kondensierte, aromatische Kohlenwasserstoffe. Oft werden Mischungen verschiedener Lösungsmittel eingesetzt, um eine optimale Löslichkeit für die Farbbildung, eine rasche und intensive Färbung und eine für die Mikroverkapselung günstige Viskosität zu erreichen.

Die Kapselwände können durch Koazervationskräfte gleichmässig um die Tröpfchen der Farbbildnerlösung herum gebildet werden, wobei das Einkapselungsmaterial z.B. aus Gelatine und Gummiarabikum bestehen kann, wie dies z.B. in der US-Patentschrift 2 800 457 beschrieben ist. Die Kapseln können vorzugsweise auch aus einem Aminoplast oder modifizierten Aminoplasten durch Polykondensation gebildet werden, wie es in den britischen Patentschriften 989,264, 1 156 725, 1 301 052 und 1 355 124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten, d.h. der Entwickler und durch das Trägermaterial. Bevorzugt wird eine Anordnung, bei der der eingekapselte Farbbildner in Form einer Schicht auf der Rückseite eines Uebertragungsblattes und der erfindungsgemäss zu verwendende Entwickler in Form einer Schicht auf der Vorderseite eines Empfangsblattes vorhanden sind.

Die Komponenten können aber auch in der Papierpulpe verwendet werden.

Eine andere Anordnung der Bestandteile besteht darin, dass die Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummiarabicum, Polyvinylalkohol, Hydroxymethylcellulose, Casein, Methylcellulose, Dextrin, Stärke oder Polymerlatices.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formel (1) können auch als Entwickler in einem thermoreaktiven Aufzeichnungsmaterial verwendet werden. Dieses enthält in der Regel mindestens einen Träger, einen Farbbildner, einen Entwickler und gegebenenfalls auch ein Bindemittel.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, verwendet. Die Bilderzeugung (Markierungserzeugung) kann auch manuell mit einer erhitzten Feder erfolgen. Eine weitere Einrichtung der Erzeugung von Markierungen mittels Wärme sind Laserstrahlen.

Das thermoreaktive Aufzeichungsmaterial kann so aufgebaut sein, dass der Farbbildner in einer Bindemittelschicht gelöst oder dispergiert ist und in einer zweiten Schicht der Entwickler in dem Bindemittel gelöst oder dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Entwickler in Kontakt und es entwickelt sich sofort die erwünschte Farbe.

Die Entwickler der Formel (1) können für sich allein, als Mischungen oder in Mischung mit bekannten Entwicklern eingesetzt werden.

Bekannt sind für diesen Zweck die gleichen Entwickler, wie sie in druckempfindlichen Papieren verwendet werden, sowie auch phenolische Verbindungen, wie z.B. 4-tert.-Butylphenol, 4-Phenylphenol, 4-Hydroxydiphenyläther, α-Naphthol, β-Naphthol, 4-Hydroxybenzoesäuremethylester, 4-Hydroxyacetophenon, 2,2'-Dihydroxydiphenyl, 4,4'-Isopropylidendiphenol, 4,4'-Isopropyliden-bis-(2-methyl-phenol), 4,4'-Bis-(hydroxyphenyl)valeriansäure, Hydrochinon, Pyrogallol, Phloroglucin, p-, m-, o-Hydroxybenzoesäure, Gallussäure, 1-Hydroxy-2-naphthoesäure sowie Borsäure und organische, vorzugsweise aliphatische Dicarbonsäuren, wie z.B. Weinsäure, Oxalsäure, Maleinsäure, Zitronensäure, Citraconsäure und Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Farbbildner und der Entwickler in Wasser unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel so dass der Farbbildner mit dem Entwickler in Kontakt kommt und sich eine Farbe bilden kann. Wasserlösliche oder mindestens in

Wasser quellbare Bindemittel sind z.B. hydrophile Polymerisate, wie Polyvinylalkohol, Polyacrylsäure, Hydroxyäthylcellulose, Methylcellulose, Carboxymethylcellulose, Polyacrylamid, Polyvinylpyrrolidon, Gelatine und Stärke.

Wenn der Farbbildner und der Entwickler in zwei getrennten Schichten vorliegen, können in Wasser unlösliche Bindemittel, d.h. in nicht-polaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Aethylcellulose Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, $TiO_2$, ZnO, $CaCO_3$, unreaktive Tone oder auch organische Pigmente, wie z.B. Harnstoff-Formaldehydpolymerisate enthalten. Um zu bewirken, dass nur innerhalb eines begrenzten Temperaturbereiches die Farbe gebildet wird, können Substanzen, wie Harnstoff, Thioharnstoff, Acetamid, Acetanilid, Stearinsäureamid, Phthalsäureanhydrid, Phthalsäurenitril oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse.

In den folgenden Beispielen beziehen sich die angegebenen Prozentsätze, wenn nichts anderes angegeben ist, auf das Gewicht.

Beispiel 1: Eine Lösung von 3 g Kristallviolettlakton in 97 g partiell hydriertem Terphenyl wird in einer Lösung von 12 g Schweinehautgelatine in 88 g Wasser von 50°C emulgiert. Sodann wird eine Lösung von 12g Gummiarabicum in 88 g Wasser von 50°C zugegeben und hierauf 200 ml Wasser von 50°C zugefügt. Die erhaltene Emulsion wird in 600 g Eiswasser eingegossen und gekühlt, wobei die Koazervation bewirkt wird. Mit der dabei erhaltenen Suspension der Mikrokapseln wird ein Blatt Papier beschichtet und getrocknet.

Ein zweites Blatt Papier wird mit einer Verbindung der Formel

(11) 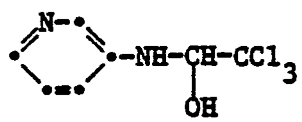

beschichtet.

Das erste Blatt und das mit der Verbindung der Formel (11) beschichtete Papier wird mit den Beschichtungen benachbart aufeinander gelegt. Durch Schreiben mit der Hand oder mit der Schreibmaschine auf dem ersten Blatt wird Druck ausgeübt und es entwickelt sich sofort auf dem mit dem Entwickler beschichteten Blatt eine intensive blaue Kopie.

Beispiel 2: In einer Kugelmühle werden 32 g einer Verbindung der Formel

(12) 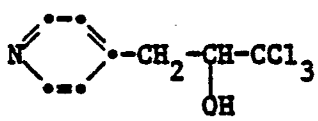 ,

3,8 g Distearylamid des Aethylendiamins, 39 g Kaolin, 20 g eines zu 88 % hydrolysierten Polyvinylalkohols und 500 ml Wasser gemahlen bis die Teilchengrösse ca. 5 µ beträgt.

In einer zweiten Kugelmühle werden 6 g 2-Phenylamino-3-methyl-6-diäthylamino-fluoran, 3 g eines zu 88 % hydrolysierten Polyvinylalkohols und 60 ml Wasser zu einer Teilchengrösse von ca. 3 µ gemahlen.

Die beiden Dispersionen werden zusammengegeben und mit einem Trockenauftragsgewicht von 5,5 $g/m^2$ auf ein Papier aufgestrichen. Durch Berührung des Papiers mit einem erhitzten Kugelschreiber wird eine intensive schwarze Farbe erhalten, die eine ausgezeichnete Lichtechtheit hat.

Auf gleiche Weise wie in den Beispielen 1 und 2 beschrieben, können auch die in der folgenden Tabelle aufgeführten Farbentwickler der Formel (13) verwendet werden.

(13) $$Z_3 - X_3 - \underset{\displaystyle OH}{\underset{|}{CH}} - CCl_3$$

| Beispiel No. | $- X_3 - Z_3$ | Smp./°C | $n_D$/°C |
|---|---|---|---|
| 3 | 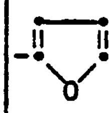 | | 1.5289/25 |
| 4 | 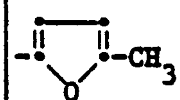 | | 1.5269/25 |
| 5 | 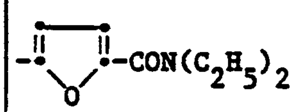 | 95-96 | |
| 6 | 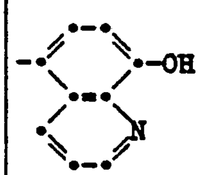 | 170-180 | |
| 7 | 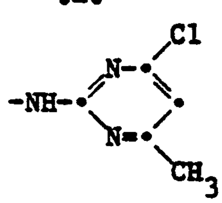 | 160-161 | |

| Beispiel No. | $-X_3-Z_3$ | Smp./°C | $n_D$/°C |
|---|---|---|---|
| 8 | 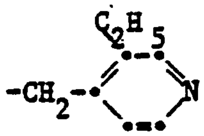 | 137 | |
| 9 | 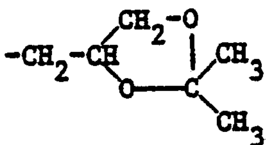 | | 1.4852/26 |
| 10 | 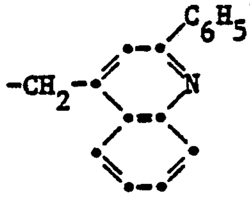 | 195 | |
| 11 | 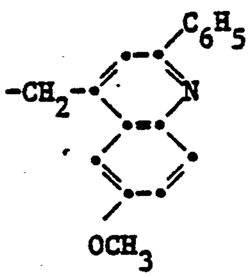 | 222 | |
| 12 | 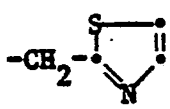 | 124-126 | |
| 13 | 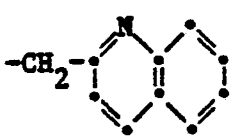 | 148 | |
| 14 | 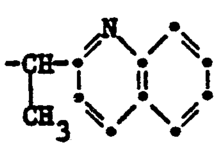 | 117-118 | |
| 15 | 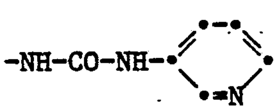 | 165-166 | |

Die Verbindung gemäss Beispiel 15 wird folgendermassen hergestellt:

Ein Gemisch von 6,85 g 3-Pyridylharnstoff (J.gen.Chem. USSR 20, 947 (1950)), 7,0 ml Chloral und 25 ml Aethylenchlorid wird 20 Stunden bei 50-55°C gerührt, worauf der entstandene Niederschlag abfiltriert wird. Nach Reinigung in siedendem Aceton und nochmaligen Abfiltrieren erhält man 8,5 g einer Verbindung der Formel

(14)

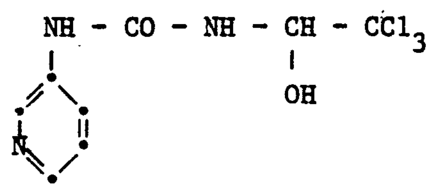

welche bei 165-166°C unter Zersetzung schmilzt.

0027913

Patentansprüche

1. Druck- oder wärmeempfindliches Aufzeichnungsmaterial, dadurch gekennzeichnet, dass es in seinem Farbreaktantensystem als Farbentwickler für den Farbbildner mindestens eine Verbindung der Formel

(1) 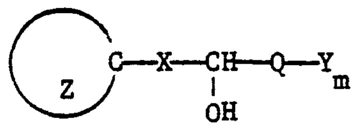

enthält, worin der ring Z einen heterocyclischen Rest darstellt, welcher benachbart zum Bindungskohlenstoffatom keine Ketogruppe aufweist, X die direkte Bindung, -O-, -CHR-, -NR-, -CONR-, $-SO_2NR-$, $-NR_1-CO-NR_2-$, $-NR_1-CS-NR_2-$ oder $-NR_1-SO_2-NR_2-$, R, $R_1$ und $R_2$ unabhängig voneinander Wasserstoff, unsubstituiertes oder durch Halogen, Cyano oder Niederalkoxy substituiertes Alkyl mit höchstens 12 Kohlenstoffatomen, Benzyl, Phenyl, oder durch Halogen, Methyl oder Methoxy substituiertes Benzyl oder Phenyl, Q Kohlenstoff oder einen unsubstituierten oder substituierten Kohlenwasserstoffrest, Y Halogen und m 1 bis 3 bedeuten.

2. Aufzeichnungsmaterial gemäss Anspruch 1, dadurch gekennzeichnet, dass der Farbentwickler der Formel (1) entspricht, worin der heterocyclische Ring Z einkernig oder mehrkernig, unsubstituiert oder durch Halogen, Cyano, Nitro, Hydroxyl, Niederalkyl, Niederalkoxy, Niederalkylcarbonyl, Niederalkoxycarbonyl, Niederalkylamino, Diniederalkylamino, N-Niederalkylcarbamyl, N,N-Diniederalkylcarbamyl oder Phenyl substituiert ist.

3. Aufzeichnungsmaterial gemäss Anspruch 1, dadurch gekennzeichnet, dass der Farbentwickler der Formel (1) entspricht, worin Q Kohlenstoff, unsubstituiertes oder Halogen, Carboxyl, $-SO_3H$, Phenyl oder Halogenphenyl substituiertes $C_1$-$C_6$-Alkylen; Cyclohexylen, Diphenylen oder unsubstituiertes oder durch Halogen, Carboxyl, $-SO_3H$, Niederalkyl oder Niederalkoxy substituiertes Phenylen bedeutet.

4. Aufzeichnungsmaterial gemäss einem der Ansprüche 1 bis 3, dadurch gekennzeichnet, dass der Farbentwickler der Formel (1) entspricht, worin Q Kohlenstoff und m 3 sind.

5. Aufzeichnungsmaterial gemäss einem der Ansprüche 1 bis 4, dadurch gekennzeichnet, dass der Farbentwickler der Formel (1) entspricht, worin X die direkte Bindung, -CHR- oder -NR- bedeutet und R die in Anspruch 1 angegebene Bedeutung hat.

6. Aufzeichnungsmaterial gemäss einem der Ansprüche 1 bis 4, dadurch gekennzeichnet, dass der Farbentwickler der Formel (1) entspricht, worin X -O-, -CONR-, $-SO_2NR-$, $-NR_1-CO-NR_2-$ oder $-NR_1-SO_2-NR_2-$ bedeutet und R, $R_1$ und $R_2$ die im Anspruch 1 angegebene Bedeutung haben.

7. Aufzeichnungsmaterial gemäss Anspruch 1, dadurch gekennzeichnet, dass der Farbentwickler der Formel

(2) 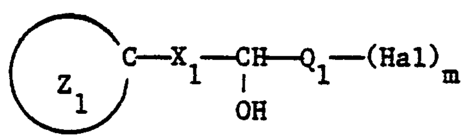

entspricht, worin der Ring $Z_1$ einen unsubstituierten oder durch Halogen, Cyano, Hydroxyl, Niederalkyl, Niederalkoxy, Niederalkylcarbonyl, Niederalkylamino, Diniederalkylamino, N-Niederalkylcarbamyl, N,N-Diniederalkylcarbamyl oder Phenyl substituierten, ein- oder zweikernigen, heterocyclischen Rest darstellt, welcher benachbart zum Bindungskohlenstoffatom keine Ketogruppe aufweist, und $X_1$ die direkte Bindung, -O-, $-CH_2-$, $-CHR_3-$, $-NR_3-$, -CONH-, $-SO_2NH-$, -NH-CO-NH- oder $-NH-SO_2-NH-$, $R_3$ Niederalkyl, $Q_1$ Kohlenstoff, $C_1-C_5$-Alkylen oder Phenylen, Hal Fluor, Chlor oder Brom und m 1 bis 3 bedeuten.

8. Aufzeichnungsmaterial gemäss Anspruch 7, dadurch gekennzeichnet, dass der Farbentwickler der Formel (2) entspricht, worin $X_1$ -O-, -CONH-, $-SO_2NH-$, -NH-CO-NH- oder $-NH-SO_2-NH-$ bedeutet.

9. Aufzeichnungsmaterial gemäss Anspruch 7, dadurch gekennzeichnet, dass der Farbentwickler der Formel

(3) 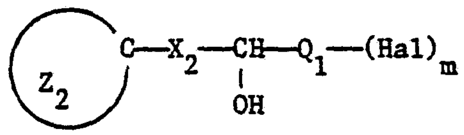

entspricht, worin der Ring $Z_2$ einen unsubstituierten oder durch Halogen, Cyano, Hydroxyl, Niederalkyl, Niederalkoxy, Niederalkylcarbonyl, N-Niederalkylcarbamyl, N,N-Diniederalkylcarbamyl oder Phenyl substituierten, ein- oder zweikernigen, heterocyclischen Rest darstellt, welcher benachbart zum Bindungskohlenstoffatom keine Ketogruppe aufweist, und $X_2$ die direkte Bindung, $-CH_2-$, $-CHR_3-$ oder -NH- bedeutet, und $R_3$, $Q_1$, Hal und m die in Anspruch 7 angegebene Bedeutung haben.

10. Aufzeichnungsmaterial gemäss Anspruch 7, dadurch gekennzeichnet, dass der Farbentwickler der Formel

(4) 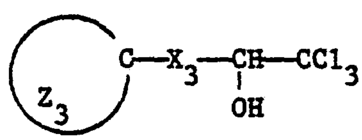

entspricht, worin der Ring $Z_3$ einen unsubstituierten oder durch Halogen, Cyano, Hydroxyl, Niederalkyl, Niederalkoxy, N-Niederalkylcarbamyl, N,N-Diniederalkylcarbamyl oder Phenyl substituierten Furyl-, Thienyl-, Pyridyl-, Pyrimidyl-, Thiazolyl- oder Chinolinylrest und $X_3$ die direkte Bindung, $-CH_2-$, -O-, -NH-, -CONH- oder -NH-CO-NH- bedeuten.

11. Aufzeichnungsmaterial gemäss Anspruch 10, dadurch gekennzeichnet, dass der Farbentwickler der Formel (4) entspricht, worin $X_3$ -O-, -CONH- oder -NH-CO-NH- und $Z_3$ einen unsubstituierten oder durch Halogen, Cyano, Hydroxyl, Niederalkyl, Niederalkoxy oder Phenyl substituierten Furyl-, Thienyl-, Pyridiyl-, Pyrimidyl-, Thiazolyl- oder Chinolinylrest bedeuten.

12. Druckempfindliches Aufzeichnungsmaterial gemäss Anspruch 1, dadurch gekennzeichnet, dass eingekapselter Farbbildner in Form einer Schicht auf der Rückseite eines Uebertragungsblattes und der Farbentwickler der Formel (1) in Form einer Schicht auf der Vorderseite des Empfangsblattes vorhanden sind.

13. Druckempfindliches Aufzeichnungsmaterial gemäss Anspruch 1, dadurch gekennzeichnet, dass die Verbindung der Formel (1) gemeinsam mit einem oder mehreren anderen Farbentwicklern enthalten ist.

14. Wärmeempfindliches Aufzeichnungsmaterial gemäss Anspruch 1, dadurch gekennzeichnet, dass es in mindestens einer Schicht mindestens einen Farbbildner, einen Farbentwickler und gegebenenfalls ein Bindemittel enthält, worin der Farbentwickler die in Anspruch 1 angegebene Formel hat.